# EUROPEAN PATENT APPLICATION

(11) **EP 2 025 647 A2**
(43) Date of publication of application: **18.02.2009**
(21) Application number: 08019390.7
(22) Date of filing: 15.09.2006
(51) Int. Cl.: C02F 3/10, C12N 11/04, C12N 11/08

(54) **Method for storing or transporting entrapping immobilization pellets**

(30) Priority: 22.09.2005 JP 2005276630
(62) Divisional of application: 06019366.1
(71) Applicant: Hitachi Plant Technologies, Ltd., Toshima-ku Tokyo (JP)
(72) Inventor: Abe, Naoki, Tokyo (JP); Aoyama, Koutarou, Tokyo (JP); Sumino, Tatsuo, Tokyo (JP)
(74) Representative: Grünecker, Kinkeldey, Stockmair & Schwanhäusser Anwaltssozietät

(57) **Abstract**

Entrapping immobilization pellets (20) in which microorganisms are entrapped and immobilized in an immobilizing agent, the immobilizing agent comprising an attachment prevention filler (22) for preventing attachment of the entrapping immobilization pellets (20). Pellets can be stored or transported inexpensively and easily without impairing their inherent pellet performance, since it is not required to add water to the storage or transportation container.

## Description

### BACKGROUND OF THE INVENTION

### Field of the Invention

The present invention relates to entrapping immobilization pellets, a process for producing the same, and a method for storing or transporting the same. More particularly, the present invention relates to entrapping immobilization pellets that can be stored or transported inexpensively and easily without impairing wastewater treatment performance, a process for producing the same, a method for storing or transporting the same, and wastewater treatment equipment.

### Description of the Related Art

Nitrifying bacteria contained in activated sludge used for wastewater treatment grow slower than common bacteria. In particular, in winter during which water temperatures are low, such nitrifying bacteria have only a small number of bacterial cells and thus exhibit significantly reduced nitrification activity. This also applies to microorganisms having the same properties as in nitrifying bacteria.

In this situation, wastewater nitrification performance of nitrifying bacteria has been improved by immobilizing activated sludge containing nitrifying bacteria to the surface of an attachment material such as quartz sand, activated carbon, or plastic to increase the concentration of nitrifying bacteria (see "Water treatment by microorganism immobilization process: Pellet immobilization process, entrapping immobilization process, biologically activated carbon process," published by NTS Inc. in 2000).

However, the concentration of nitrifying bacteria cannot be sufficiently increased in attachment immobilization pellets in which microorganisms are attached to an attachment material, because the attached microorganisms are released from the material, or microorganisms differing from nitrifying bacteria as target microorganisms are attached to the material. Accordingly, wastewater has been treated at high speed with increased nitrification activity by producing entrapping immobilization pellets in which useful microorganisms such as nitrifying bacteria are entrapped and immobilized in an immobilizing agent, and packing a reaction tank with the entrapping immobilization pellets to increase the concentration of nitrifying bacteria. Entrapping immobilization pellets are produced by mixing activated sludge with an immobilizing agent such as a polymer compound to prepare a raw material liquid, and polymerizing the raw material liquid using a polymerization initiator to form a gel (for example, Japanese Patent No. 3,422,229).

Entrapping immobilization pellets of microorganisms thus produced are stored or transported in a bag or case together with a slight small of water. In wastewater treatment equipment, the entrapping immobilization pellets are removed from the bag or case, introduced into a biological treatment tank, and used for wastewater treatment.

However, an unpolymerized matter of the immobilizing agent may remain on the surface of entrapping immobilization pellets produced as described above (hereinafter referred to as pellets) depending on the production conditions. Thus, when the surface of pellets during storage or transportation is dried, the unpolymerized matter on the surface of the pellets often has adhesiveness to cause the pellets to adhere to each other, thereby forming a ball-like mass. For this reason, even if the mass is introduced into a biological treatment tank and brought into contact with water, the pellets once made into a mass are separated only with difficulty and cannot exhibit their inherent pellet performance, disadvantageously.

Therefore, when pellets are stored or transported, the pellets are introduced into a bag or case together with water to prevent the pellets from directly adhering to each other and forming a mass. However, a drawback in this method is much labor and high cost, because a bag or case for storing pellets has an increased volume and thus is bulky and has an increased weight. Another drawback in this method is that water introduced into a bag or case is rotted and produces offensive odor. For this reason, it has been demanded that pellets can be stored or transported without formation of a mass even if water is not introduced into a bag or case.

The present invention has been achieved in view of such circumstances. An object of the present invention is to provide entrapping immobilization pellets that can be stored or transported easily and inexpensively without impairing their inherent pellet performance, a process for producing the same, a method for storing or transporting the same, and wastewater treatment equipment.

### SUMMARY OF THE INVENTION

To attain the aforementioned object, according to a first aspect of the present invention, there is provided entrapping immobilization pellets in which microorganisms are entrapped and immobilized in an immobilizing agent, the immobilizing agent comprising an attachment prevention filler for preventing attachment of the entrapping immobilization pellets.

According to the first aspect, an attachment prevention filler is added when microorganisms are entrapped and immobilized in an immobilizing agent. The present inventors have known that it is difficult to adhere pellets to each other if a specific attachment prevention filler is added and polymerization is carried out during entrapping immobilization of microorganisms in an immobilizing agent, and the inventors have found a method for storing or transporting pellets without introduction of water based on this knowledge.

Accordingly, pellets can be stored or transported easily and inexpensively without impairing their inherent pellet performance.

The attachment prevention filler is preferably talc, fly ash, powdered activated carbon, or calcium carbonate.

According to a second aspect of the present invention, there is provided the entrapping immobilization pellets according to the first aspect, wherein the attachment prevention filler is an inorganic filler.

An organic filler is not preferably used for entrapping and immobilizing microorganisms in an immobilizing agent, because the organic filler is easily decomposed by microorganisms or the like and cannot exist as particles on the surface of the pellets. According to the second aspect, an inorganic filler is added when microorganisms are entrapped and immobilized. Thus, the inorganic filler can exist as particles on the surface of the pellets after polymerization without dissolution in the immobilizing agent, and can prevent the pellets from adhering to each other. Accordingly, pellets can be stored or transported easily and inexpensively without impairing their inherent pellet performance. In the second aspect, the inorganic filler is preferably talc, fly ash, or calcium carbonate.

According a third aspect of the present invention, there is provided the entrapping immobilization pellets according to the first or second aspect, wherein the attachment prevention filler has an average particle size of 5 to 100 µm.

According to the third aspect, the pellets adhere to each other only with difficulty when the attachment prevention filler has an average particle size of 5 to 100 µm. Accordingly, pellets can be stored or transported easily and inexpensively without impairing their inherent pellet performance.

Specifically, particles of the attachment prevention filler are too small and uniformly dispersed only with difficulty when the attachment prevention filler has an average particle size of less than 5 µm, and on the other hand, the area of the pellet surface in contact with the attachment prevention filler is small when the attachment prevention filler has an average particle size of more than 100 µm. Therefore, the attachment prevention filler immobilized on the pellets is easily dropped out, unfavorably, and the pellet strength tends to be decreased, unfavorably, because cracks easily occur in the pellets. The average particle size is a particle size of a sphere having the same volume as that of the attachment prevention filler.

According to a fourth aspect of the present invention, there is provided the entrapping immobilization pellets according to any one of the first to third aspects, wherein the attachment prevention filler has a concentration of 3 to 20 mass% based on the entrapping immobilization pellets.

According to the fourth aspect, the present inventors have found that the pellets adhere to each other only with difficulty when the concentration of the attachment prevention filler is 3 to 20 mass%. Accordingly, pellets can be stored or transported easily and inexpensively without impairing their inherent pellet performance.

According to a fifth aspect of the present invention, there is provided the entrapping immobilization pellets according to any one of the first to fourth aspects, wherein the immobilizing agent comprises an acrylate derivative or diacrylate derivative of a polymer comprised of ethylene oxide and propylene oxide, and the acrylate derivative or diacrylate derivative has a molecular weight of 1,000 to 10,000.

Since the immobilizing agent of the fifth aspect is water-soluble and has a relatively low viscosity, the attachment prevention filler is easily uniformly dispersed in the immobilizing agent. Thus, the attachment prevention filler is uniformly dispersed on the surface of the pellets. Accordingly, pellets adhere to each other only with difficulty, and can be stored or transported easily and inexpensively without impairing their inherent pellet performance.

According to a sixth aspect of the present invention, there is provided the entrapping immobilization pellets according to any one of the first to fifth aspects, wherein the attachment prevention filler is located on a part of the surface of the entrapping immobilization pellets.

According to the sixth aspect, a large amount of the attachment prevention filler is located on a part of the surface of the entrapping immobilization pellets. Therefore, the pellets adhere to each other only with difficulty even if only a small amount of the attachment prevention filler is added. Accordingly, pellets can be stored or transported easily and inexpensively without impairing their inherent pellet performance.

To attain the aforementioned object, according to a seventh aspect of the present invention, there is provided a process for producing the entrapping immobilization pellets of the sixth aspect, the process comprising spreading in advance an attachment prevention filler over the internal surface of a forming container or over a forming sheet; casting a liquid mixture of at least microorganisms and an immobilizing agent on the attachment prevention filler; polymerizing the liquid mixture to prepare a formed product; and cutting the formed product into pellets.

According to the process for producing entrapping immobilization pellets of the seventh aspect, an attachment prevention filler can be located on a part of the surface of the pellets and immobilized. Accordingly, pellets can be stored or transported easily and inexpensively without impairing their inherent pellet performance. In the seventh aspect, entrapping immobilization pellets are suitably produced by tube forming, drop granulation, sheet forming, or the like. Examples of the forming container or forming sheet include, but are not limited to, tube-shaped or block-shaped forming containers and sheet-shaped conveyors (such as belt conveyors) which can gel (or form) a liquid mixture.

To attain the aforementioned object, according to an eighth aspect of the present invention, there is provided a method for storing or transporting the entrapping immobilization pellets of any one of the first to sixth aspects in a storage container, the method comprising storing or transporting the entrapping immobilization pellets without introducing water into the storage container.

According to the eighth aspect, pellets can be stored or transported without introducing water into a bag or case, unlike a conventional storage or transportation method in which water is introduced into a bag or case. Specifically, the present inventors have known that pellets obtained by polymerization after addition of a specific attachment prevention filler adhere to each other only with difficulty, and the inventors have found that pellets can be directly introduced alone into a bag or case for storage or transportation, even if water is not introduced thereinto. Accordingly, pellets can be stored or transported inexpensively and easily.

According to a ninth aspect of the present invention, there is provided wastewater treatment equipment in which the entrapping immobilization pellets of any one of the first to sixth aspects are used.

In the ninth aspect, the entrapping immobilization pellets of the present invention are used in wastewater treatment equipment. According to the ninth aspect, since the entrapping immobilization pellets of the present invention are introduced into a biological treatment tank, the biological treatment tank can be packed with microorganisms at a high concentration without impairing pellet performance, and wastewater can be efficiently treated.

As described above, according to the present invention, pellets can be stored or transported inexpensively and easily without impairing their inherent pellet performance.

### BRIEF DESCRIPTION OF THE DRAWINGS

Fig. 1 is a view describing a first embodiment of the process for producing entrapping immobilization pellets of the present invention;
Figs. 2A to 2C are schematic views showing the effect of conventional entrapping immobilization pellets;
Figs. 3A to 3B are schematic views showing the effect of the entrapping immobilization pellets of the present embodiment;
Figs. 4A to 4B are schematic views showing the method for storing or transporting entrapping immobilization pellets of the present embodiment;
Fig. 5 is a schematic view showing an outline of an apparatus for producing entrapping immobilization pellets of another embodiment;
Fig. 6 is a view for showing an operation flow in a process for producing entrapping immobilization pellets of another embodiment;
Fig. 7 is a graph showing the results for the present example; and
Fig. 8 is a graph showing the results for the present example.

### DETAILED DESCRIPTION OF THE PREFERRED EMBODIMENTS

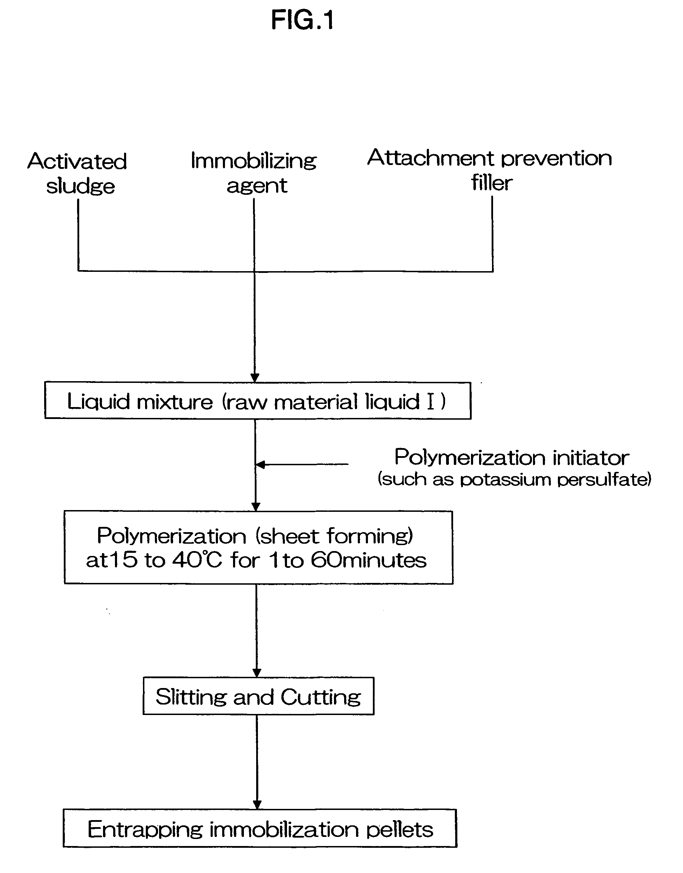

Preferred embodiments of the entrapping immobilization pellets, the process for producing the same, the method for storing or transporting the same, and wastewater treatment equipment of the present invention will be described in detail below with reference to the accompanying drawings. Fig. 1 is a view describing a first embodiment of the process for producing entrapping immobilization pellets of the present invention,
in which an attachment prevention filler is dispersed in the whole pellets and the mixture is polymerized to form a gel.

As shown in Fig. 1, activated sludge containing microorganisms, an immobilizing agent, and an attachment prevention filler are mixed first to prepare a raw material liquid I. In order to improve dispersibility of the attachment prevention filler in the immobilizing agent and reduce adhesiveness of the entrapping immobilizing pellets to each other, the attachment prevention filler has an average particle size of 5 to 100 µm and is added in an amount of 3 to 20 mass%. Next, a polymerization initiator such as potassium persulfate is added to the raw material liquid I, and the mixture is polymerized (gelled) at a polymerization temperature of 15 to 40°C, and preferably 20 to 30°C, for a polymerization time of 1 to 60 minutes, and preferably 1.5 to 60 minutes. Then, the gelled pellet sheet is cut into about 3 mm-square angular pellets. The entrapping immobilization pellets of the present invention are thus produced.

As the microorganisms, activated sludge containing complex microorganisms such as nitrifying bacteria, denitrifying bacteria, or anaerobic ammonium oxidizing bacteria may be used. In order to increase the initial immobilizing concentration of the target microorganisms, the activated sludge concentration is preferably 10,000 to 40,000 mg-ss/L. The microorganisms are not limited to activated sludge. Pure microorganisms such as nitrifying bacteria, denitrifying bacteria, anaerobic ammonium oxidizing bacteria, BOD component oxidizing bacteria, bisphenol A decomposing bacteria, microcystis decomposing bacteria, PCB decomposing bacteria, dioxin decomposing bacteria, and environmental hormone decomposing bacteria may be used.

Examples of the attachment prevention filler that may be used include talc, plate-like alumina, kaolinite, Sillitin, Aktisil, a mica powder, a zinc oxide whisker, wollastonite, potassium titanate, a magnesium sulfate whisker, a calcium silicate whisker, mica, synthetic mica, a graphite powder, a carbon fiber, fly ash, powdered activated carbon, calcium carbonate, calcium sulfate, calcium sulfite, magnesium hydroxide, aluminum hydroxide, antimony oxide, zinc stannate, titanium oxide, zinc oxide, a silica powder, glass beads, diatomite, calcium silicate, attapulgite, asbestos, carbon black, acetylene black, furnace black, white carbon, pyrophyllite clay, silica, cotton, polyester, nylon, silicon nitride, molybdenum disulfide, iron oxide, basic magnesium carbonate, hydrotalcite, alumina, zirconium oxide, bentonite, zeolite, kaolin clay, sericite, zirconium silicate, barium sulfate, barium carbonate, barium titanate, zinc oxide, kaolin, sepiolite, smectite, and vermiculite.

The immobilizing agent may be a high-molecular-weight monomer, prepolymer, oligomer, or the like, but is not specifically limited. For example, a polyethylene glycol polymer, a polyvinyl alcohol polymer, or the like is preferably used. Specifically, a prepolymer having a molecular weight of 4,000 which contains ethylene oxide and propylene oxide at 7:3 and has a diacrylate as a terminal group may be preferably used.

Further, polyethylene glycol acrylate, polyethylene glycol diacrylate, polyethylene glycol methacrylate, or the like may be used as the immobilizing agent. In addition, the following prepolymers may also be used:
monomethacrylates such as polyethylene glycol monomethacrylate, polyprene glycol monomethacrylate, polypropylene glycol monomethacrylate, methoxydiethylene glycol methacrylate, methoxypolyethylene glycol methacrylate, methacryloyloxyethyl hydrogen phthalate, methacryloyloxyethyl hydrogen succinate, 3-chloro-2-hydroxypropyl methacrylate, stearyl methacrylate, 2-hydroxy methacrylate, and ethyl methacrylate; monoacrylates such as 2-hydroxyethyl acrylate, 2-hydroxypropyl acrylate, isobutyl acrylate, t-butyl acrylate, isooctyl acrylate, lauryl acrylate, stearyl acrylate, isobornyl acrylate, cyclohexyl acrylate, methoxytriethylene glycol acrylate, 2-ethoxyethyl acrylate, tetrahydrofurfuryl acrylate, phenoxyethyl acrylate, nonylphenoxypolyethylene glycol acrylate, nonylphenoxypolypropylene glycol acrylate, silicon-modified acrylate, polypropylene glycol monoacrylate, phenoxyethyl acrylate, phenoxydiethylene glycol acrylate, phenoxypolyethylene glycol acrylate, methoxypolyethylene glycol acrylate, acryloyloxyethyl hydrogen succinate, and lauryl acrylate;
dimethacrylates such as 1,3-butylene glycol dimethacrylate, 1,4-butanediol dimethacrylate, ethylene glycol dimethacrylate, diethylene glycol dimethacrylate, triethylene glycol dimethacrylate, polyethylene glycol dimethacrylate, butylene glycol dimethacrylate, hexanediol dimethacrylate, neopentyl glycol dimethacrylate, polyprene glycol dimethacrylate, 2-hydroxy-1,3-dimethacryloxypropane, 2,2-bis-4-methacryloxyethoxyphenylpropane, 3,2-bis-4-methacryloxydiethoxyphenylpropane, and 2,2-bis-4-methacryloxypolyethoxyphenylpropane;
diacrylates such as ethoxylated neopentyl glycol diacrylate, polyethylene glycol diacrylate, 1,6-hexanediol diacrylate, neopentyl glycol diacrylate, tripropylene glycol diacrylate, polypropylene glycol diacrylate, 2,2-bis-4-acryloxyethoxyphenylpropane, 2-hydroxy-1-acryloxy-3-methacryloxypropane;
trimethacrylates such as trimethylolpropane trimethacrylate;
triacrylates such as trimethylolpropane triacrylate, pentaerythritol triacrylate, trimethylolpropane EO-added triacrylate, glycerol PO-added triacrylate, and ethoxylated trimethylolpropane triacrylate;
tetraacrylates such as pentaerythritol tetraacrylate, ethoxylated pentaerythritol tetraacrylate, propoxylated pentaerythritol tetraacrylate, and ditrimethylolpropane tetraacrylate;
urethane acrylates such as urethane acrylate, urethane dimethyl acrylate, and urethane trimethyl acrylate; and
other compounds such as acrylamide, acrylic acid, and dimethylacrylamide.

In the present embodiment, polymerization of the entrapping immobilization pellets is most appropriately radical polymerization using potassium persulfate, but may be polymerization using ultraviolet rays or electron beams or redox polymerization. In radical polymerization using potassium persulfate, potassium persulfate is preferably added in an amount of 0.001 to 0.25 mass%, and an amine polymerization accelerator is preferably added in an amount of 0.01 to 0.5 mass% as β-dimethylaminopropionitrile, N,N,N',N'-tetramethylethylenediamine, or the like.

In the present embodiment, sheet forming is used as a method for forming entrapping immobilization pellets, but the method is not limited thereto. Tube forming, drop granulation, block forming, or the like may also be used.

Next, the main effect of the entrapping immobilization pellets of the present invention produced as described above will be described.

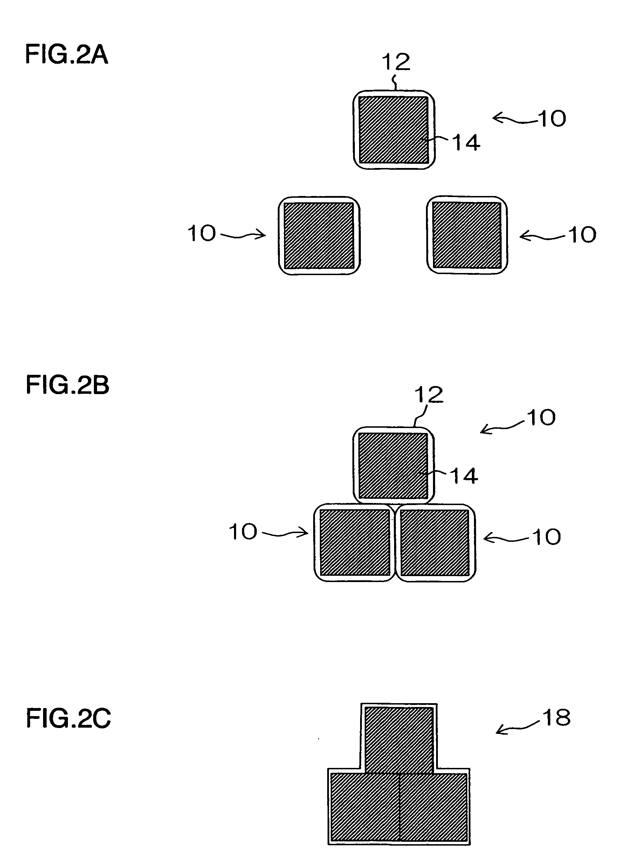

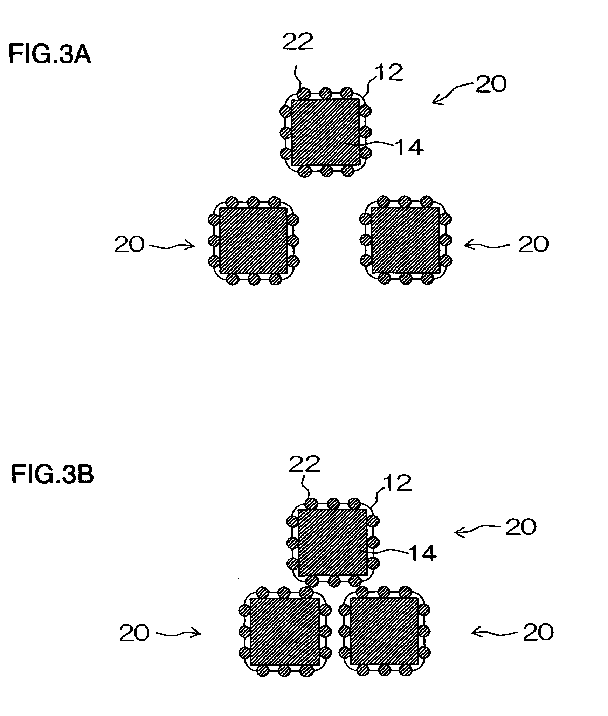

Figs. 2A to 2C are schematic views showing the main effect of conventional entrapping immobilization pellets 10. Figs. 3A to 3B are schematic views showing the main effect of entrapping immobilization pellets 20 of the present invention. An attachment prevention filler 22 in the entrapping immobilization pellets 20 is not shown in Figs. 3A to 3B.

As shown in Fig. 2A, an unpolymerized matter 12 exists on the surface of the conventional entrapping immobilization pellets 10 (hereinafter referred to as pellets 10) (the reference numeral 14 denotes a polymerized matter). Here, when the surface of the pellets 10 is dried, the unpolymerized matter 12 remaining on the surface of the pellets 10 has adhesiveness. Then, when a plurality of the pellets 10... are brought into contact with each other as shown in Fig. 2B, the unpolymerized matters 12, 12 on the surface of the pellets 10 are brought into contact with each other, and adhere to each other to form a ball-like mass 18 as shown in Fig. 2C.

On the other hand, as shown in Fig. 3A, an attachment prevention filler 22 is dispersed and immobilized in an unpolymerized matter 12 on the surface of entrapping immobilization pellets 20 of the present invention (hereinafter referred to pellets 20). Here, even if a plurality of the pellets 20... are brought into contact with each other as shown in Fig. 3B, the attachment prevention fillers 22, 22 on the pellets 20 are brought into contact with each other, or the attachment prevention filler 22 on one pellet is brought into contact with the unpolymerized matter 12 on another pellet, unlike conventional entrapping immobilization pellets. Thus, it is difficult to bring the unpolymerized matters 12, 12 on the surface of the pellets 20 into direct contact with each other, and to form a ball-like mass. The attachment prevention filler 22 on the surface of the pellets 20 is immobilized by the unpolymerized matter 12 surrounding the filler, and thus is dropped out of the surface of the pellets 20 only with difficulty.

Further, as a method for making an attachment prevention filler present on the surface of pellets, a conventional method of sprinkling an attachment prevention filler on the surface of the pellets seems to be effective. However, this method is not suitable for mass production, because not only does the method require much labor of the operator, but also a part of the attachment prevention filler is not attached to the surface of the pellets and thus more than an optimal amount of the attachment prevention filler is needed.

The effect of the pellets 20 of the present invention is as described above. However, the effect of the present invention is not simply based on such an effect.

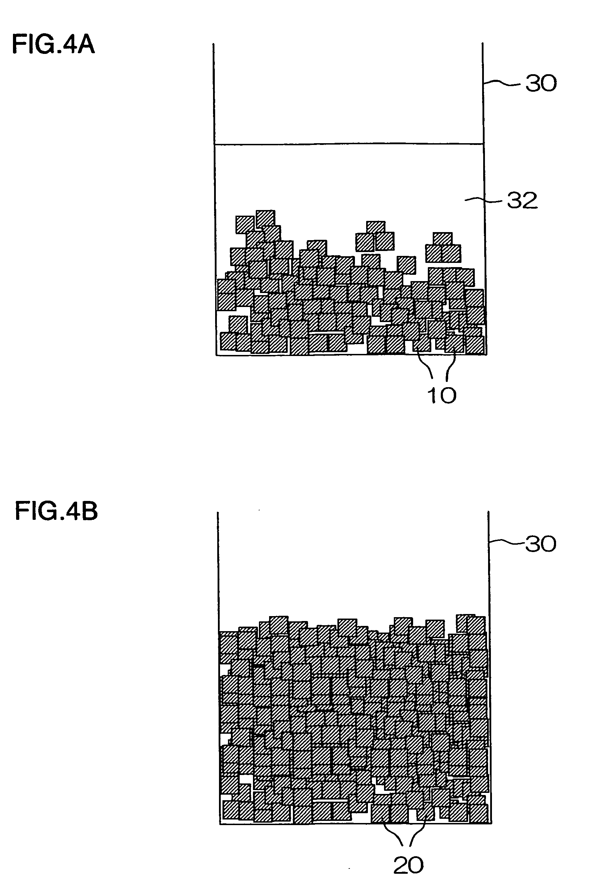

Next, a method for storing or transporting entrapping immobilization pellets utilizing the effect of the entrapping immobilization pellets of the present invention will be described. Figs. 4A to 4B are schematic views showing a method for storing entrapping immobilization a pellet, in which Fig. 4A is a view showing a method for storing conventional pellets 10 and Fig. 4B is a view showing a method for storing pellets 20 of the present invention.

As shown in Fig. 4A, in order to prevent direct mutual contact and mutual adhesion of the pellets 10..., the conventional pellets 10 are dipped in water 32 in a storage case 30 and stored. Here, when the storage temperature is high, in particular, the water 32 is rotted and produces offensive odor. This is mainly because an organic substance or an attached organic substance (such as an unpolymerized matter 12) is eluted from the pellets 10 to the water 32 and consequently microorganisms are proliferated in the water 32. For this reason, the storage case 32 containing the pellets 10 and the water 32 is generally stored or transported in a low-temperature environment.

On the other hand, as shown in Fig. 4B, the pellets 20 of the present invention are directly introduced into a storage case 30 into which water is not introduced and stored, because the pellets 20 adhere to each other only with difficulty even in a dry environment. Here, since water is not introduced into the storage tank 30, there is no increase in the volume or weight of the pellets 20 that can be stored in the storage tank 30, and it is not necessary to store the storage case 30 in a low temperature environment. Thus, efficiency in storing or transporting pellets can be drastically increased.

Here, the storage case 30 is preferably made of a resin, metal, vinyl, or the like, each of which does not react with pellets 20.

Then, the pellets 20 of the present invention are added to a biological treatment tank in a wastewater treatment system and used for biological treatment (for example, nitrification treatment) of raw water. Since the outlet of the biological treatment tank is provided with a screen, the pellets are prevented from being discharged and are retained in the biological treatment tank in a stable manner. Accordingly, a biological treatment tank to which the pellets 20 of the present invention are applied can maintain high biological treatment performance.

In the embodiment of the present invention described above, when microorganisms are entrapped and immobilized by an immobilizing agent, a specific attachment prevention filler is added thereto, and the mixture is polymerized, the resulting pellets adhere to each other only with difficulty.

Therefore, pellets can be stored or transported inexpensively and easily without impairing their inherent pellet performance.

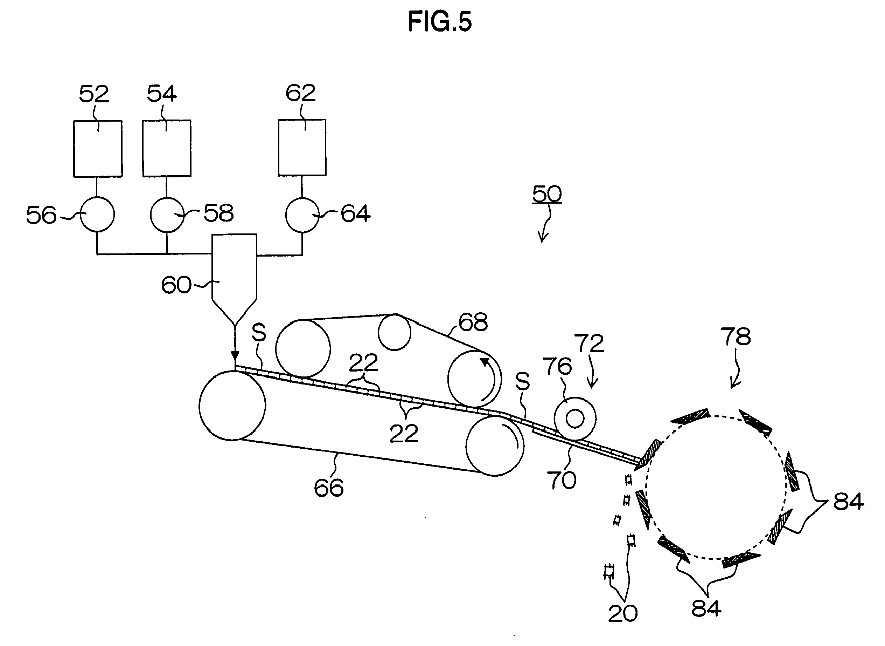

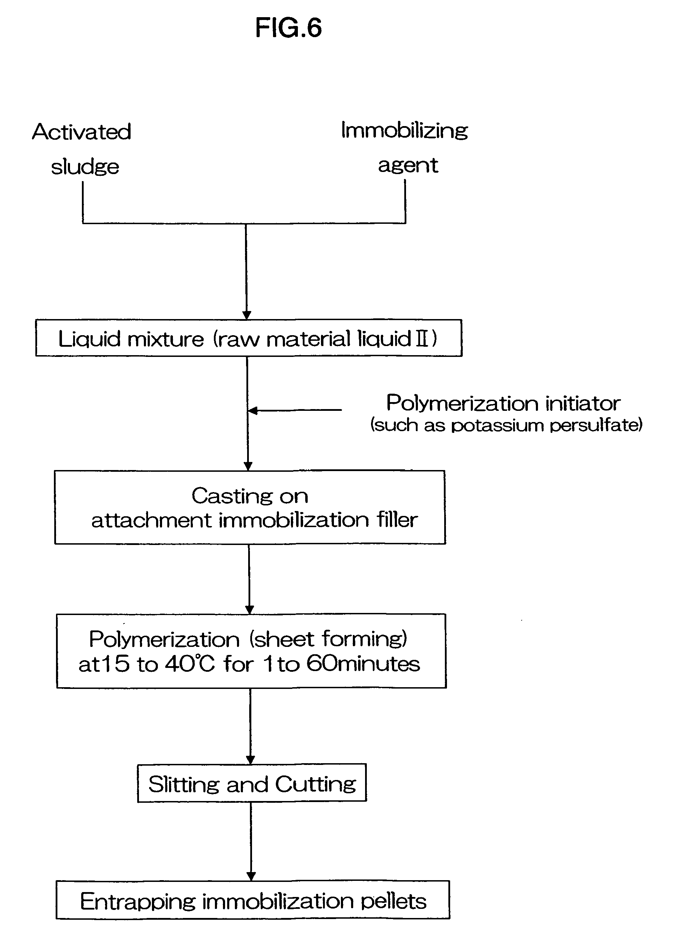

Next, a second embodiment of the process for producing entrapping immobilization pellets of the present invention will be described with reference to Figs. 5 and 6. This embodiment relates to a process for producing pellets in which an attachment prevention filler is located on a part of a part of the surface of the pellets. The present embodiment is the same as the first embodiment, except that the attachment prevention filler is located on a part of the pellets. Detailed description of the same parts is omitted.

Fig. 5 is a side view showing an outline of an apparatus 50 for producing entrapping immobilization pellets. Fig. 6 is a view describing an operation flow of the above-described pellet production process.

As shown in Fig. 5, the pellet production apparatus 50 comprises, as main components, a raw material tank 52 for storing a raw material, chemical tanks 54 and 62 for storing chemicals, a stirring and extrusion unit 60, belt conveyors 66 and 68, a slitting unit 76, and a cutting unit 78.

The raw material tank 52 stores activated sludge or the like containing microorganisms. The chemical tank 54 stores an immobilizing agent or the like. The chemical tank 62 stores a polymerization initiator or the like.

The belt conveyors 66 and 68 polymerize a raw material liquid II extruded from the stirring and extrusion unit 60 (see Fig. 6) while conveying the liquid to form a sheet. A predetermined amount of an attachment prevention filler 22 is spread over the surface of the belt conveyors 66 and 68. This may be realized, however not exclusively, by a method in which the attachment prevention filler 22 is attached to the belt conveyors 66 and 68 having adhesiveness to the extent that polymerization is not inhibited.

Here, the attachment prevention filler 22 is added to the surface of the pellet sheet S preferably in an amount of 3 to 20 mass%.

Next, the process for producing pellets 20 of the present embodiment will be described using the production apparatus 50.

First, activated sludge or the like in the raw material tank 52 and an immobilizing agent in the chemical tank 54 are mixed and, at the same time, fed to the stirring and extrusion unit 60 (raw material liquid II) by driving pumps 56 and 58. The raw material liquid II fed to the stirring and extrusion unit 60 is mixed with a polymerization initiator or the like fed from the other chemical tank 62 by a pump 64. The mixture was stirred and then extruded onto the running belt conveyor 66. Here, the raw material liquid II is polymerized and gelled while being held and conveyed by the belt conveyors 66 and 68 over which the attachment prevention filler 22 is spread. Thus, the attachment prevention filler 22 is immobilized uniformly on the surface of the pellet sheet S in contact with the belt conveyors 66 and 68. The continuous pellet sheet S in which the attachment prevention filler 22 is dispersed on both sheet surfaces is formed in this manner.

The pellet sheet S is extruded on a receiver plate 70 from the belt conveyor 66, and then slit into about 3 mm-wide lattices by a slitting unit 72 that is equipped with a circular blade 76 rotated in the conveying direction. Thereafter, the pellet sheet S is cut and pelletized into about 3 mm-square angular pellets 20 by a cutting unit 78 that is equipped with a rotary blade 84 perpendicular to the conveying direction. The pellets 20 of the present embodiment are produced in this manner.

In the embodiment of the present invention described above, since an attachment prevention filler is located on a part of a part of the pellet surface where the pellets are to adhere to each other, only a small amount of the attachment prevention filler added can prevent adhesion of the pellets to each other.

Therefore, pellets can be stored or transported inexpensively and easily without impairing their inherent pellet performance. Example Next, the present invention will be described in more detail below with reference to an example. However, the present invention is not limited to the following example.

In the present embodiment, materials shown in Table 1 were used for pellets 20. The pellets 20 were produced according to the above-described operation flow in Fig. 1.

**[Table 1]**

| Type of material | Content |
|---|---|
| Activated sludge | MLSS 30,000 mg/L |
| Immobilizing agent | Polyethylene glycol methacrylate: 10 mass% |
| Attachment prevention filler | Powdered activated carbon |
| Polymerization initiator | Potassium persulfate: 0.025 mass% |
| Polymerization accelerator | N,N,N',N'-tetramethylethylenediamine: 0.05 mass% |

### 1) Relation between amount of attachment prevention filler and mutual adhesiveness of pellets

The activated sludge, the immobilizing agent, and various amounts of the attachment prevention filler 22 (in which powdered activated carbon having an average particle size of 50 µm was used) shown in Table 1 were mixed to prepare a raw material liquid I. Then, the polymerization initiator shown in Table 1 was added to the raw material liquid I, the mixture was polymerized at a polymerization temperature of 20°C for 0.5 hour, and an about 10 cm-square pellet sheet S is formed. Next, the pellet sheet S was cut and divided to produce 1 cm-square substantially cubic entrapping immobilization pellets 20.

The amount of the attachment prevention filler 22 added at this time was changed within the range of 0 to 20 mass% based on the mass of the pellets, and the relation between the amount of the attachment prevention filler and mutual adhesiveness of the pellets was evaluated.

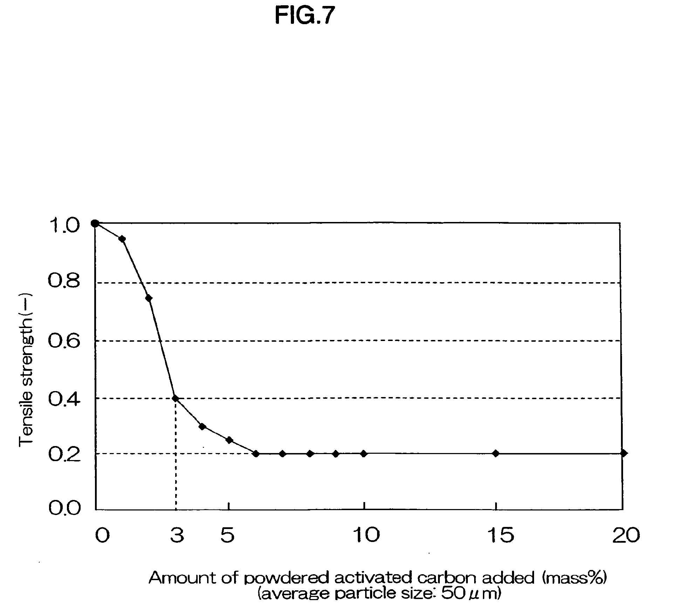

Mutual adhesiveness of the pellets was evaluated as follows. First, two pellets of the present invention were dried in a drier (105°C) for one hour to allow the surface of the pellets to have adhesiveness. Then, the two pellets were brought into contact with each other and allowed to stand for 72 hours to cause the pellets to adhere to each other. Next, the strength enough to separate the adhered pellets (tensile strength) was measured by a rheometer. Here, the strength per unit area immediately before separation of the pellets 20 with each other when the pellets were drawn at a certain force was defined as tensile strength, and was represented as a relative value with respect to the tensile strength for pellets with an attachment prevention filler not added as "1". The measurement results are shown in Fig. 7.

As shown in Fig. 7, the tensile strength was 0.4 or less when the attachment prevention filler was added in an amount of 3 to 20 mass% based on the mass of the pellets; the tensile strength was reduced to 0.2 or less and almost constant when the attachment prevention filler was added in an amount of 5 to 20 mass%.

Accordingly, it was found that the pellets adhere to each other only with difficulty and the effect of the present invention can be achieved well when the attachment prevention filler is added in an amount of 3 to 20 mass%, and preferably 5 to 20 mass% based on the mass of the pellets.

### 2) Relation between average particle size of attachment prevention filler and mutual adhesiveness of pellets

Entrapping immobilization pellets 20 were produced in the same manner as in the above 1), except that an attachment prevention filler 22 (powdered activated carbon) was added in a fixed amount of 5 mass%, and the average particle size of the attachment prevention filler 22 was changed within the range of 3 to 100 µm.

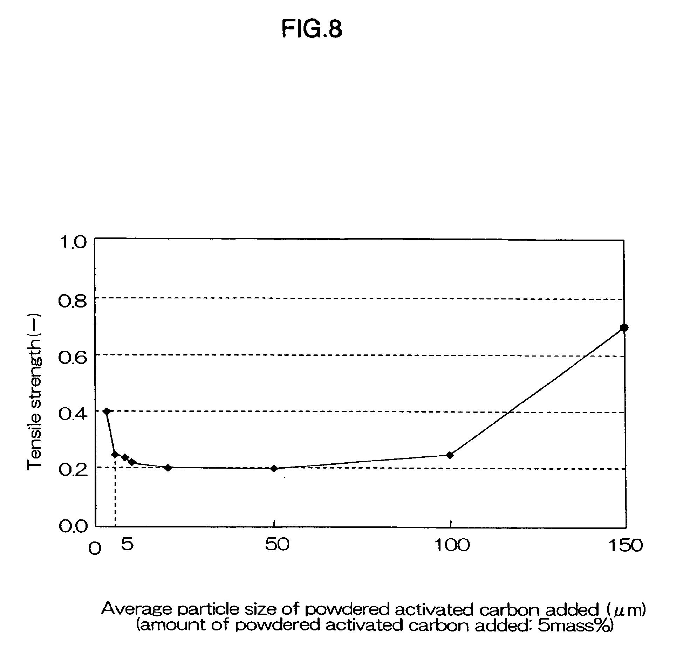

The relation between the average particle size of the attachment prevention filler and mutual adhesiveness of the pellets was evaluated in the same manner as in the above 1). The measurement results are shown in Fig. 8.

As shown in Fig. 8, the tensile strength was 0.4 or less when the attachment prevention filler had an average particle size of 3 to 100 µm; the tensile strength was reduced to 0.2 or less and almost constant when the attachment prevention filler had an average particle size of 5 to 100 µm. However, the tensile strength was considerably increased when the attachment prevention filler had an average particle size of more than 100 µm. This is presumably because the attachment prevention filler having an average particle size of more than 100 µm was easily dropped out from the surface of the pellets to cause the pellets to adhere to each other easily.

On the other hand, the tensile strength was slightly increased when the attachment prevention filler had an average particle size of less than 5 µm. This is presumably because the attachment prevention filler having a too small average particle size is uniformly dispersed in the immobilizing agent only with difficulty and forms spheres easily, and mutual adhesiveness of the pellets is increased.

Accordingly, it was found that the pellets adhere to each other only with difficulty and the effect of the present invention can be achieved well when the attachment prevention filler has an average particle size of 3 to 100 µm, and preferably 5 to 100 µm.

## Claims

1. A method for storing or transporting entrapping immobilization pellets (20) in a storage container (30), the method comprising the steps of:
storing or transporting the entrapping immobilization pellets (20) without introducing water into the storage container (30), wherein the entrapping immobilization pellets (20) are prepared by a process comprising the steps of:
spreading in advance an attachment prevention filler (22) over the internal surface of a forming container or over a forming sheet (66);
casting a liquid mixture of at least microorganisms and an immobilizing agent on the attachment prevention filler (22);
polymerizing the liquid mixture to prepare a formed product; and
cutting the formed product into pellets.

2. The method according to claim 1, wherein the attachment prevention filler (22) is an inorganic filler.

3. The method according to claim 1 or 2, wherein the attachment prevention filler (22) has an average particle size of 5 to 100 µm.

4. The method according to any one of claims 1 to 3, wherein the attachment prevention filler (22) has a concentration of 3 to 20 mass% based on the entrapping immobilization pellets (20).

5. The method according to any one of claims 1 to 4, wherein the immobilizing agent comprises an acrylate derivative or diacrylate derivative of a polymer comprised of ethylene oxide and propylene oxide, and the acrylate derivative or diacrylate derivative has a molecular weight of 1,000 to 10,000.
